Europäisches Patentamt

European Patent Office

Office européen des brevets

(11) Publication number: **0 444 505 B1**

# EUROPEAN PATENT SPECIFICATION

(45) Date of publication of patent specification: **03.05.95**   (51) Int. Cl.6: **C07C 2/32**, C07C 11/02

(21) Application number: **91102385.1**

(22) Date of filing: **20.02.91**

(54) **Process of producing linear alpha-olefins.**

(30) Priority: **28.02.90 JP 45694/90**

(43) Date of publication of application:
**04.09.91 Bulletin 91/36**

(45) Publication of the grant of the patent:
**03.05.95 Bulletin 95/18**

(84) Designated Contracting States:
**BE CH DE FR GB IT LI NL SE**

(56) References cited:
**EP-A- 0 241 596**
**EP-A- 0 320 571**
**US-A- 4 442 309**

(73) Proprietor: **IDEMITSU PETROCHEMICAL CO.
LTD.**
**1-1, Marunouchi 3-chome**
**Chiyoda-ku**
**Tokyo 100 (JP)**

(72) Inventor: **Shiraki, Yasushi**
**c/o IDEMITSU PETROCHEMICAL CO., LTD.,**
**No. 1-1**
**Singuu-cho,**
**Tokuyama-shi,**
**Yamaguchi-ken (JP)**
Inventor: **Ueda, Kenichi**
**c/o IDEMITSU PETROCHEMICAL CO., LTD.,**
**No. 1-1**
**Singuu-cho,**
**Tokuyama-shi,**
**Yamaguchi-ken (JP)**
Inventor: **Takeuchi, Kunio**
**c/o IDEMITSU PETROCHEMICAL CO., LTD.,**
**No. 1-1**
**Singuu-cho,**
**Tokuyama-shi,**
**Yamaguchi-ken (JP)**

(74) Representative: **Türk, Gille, Hrabal, Leifert**
**Brucknerstrasse 20**
**D-40593 Düsseldorf (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

1. Field of the Invention

The present invention relates to a process of producing linear α-olefins and, more particularly, relates to a process of producing linear α-olefins useful as raw materials for high molecular polymer, plasticizer, surface active agent, etc., which comprises an ash removing step by which a catalyst and its metal components can be efficiently removed to a very low concentration in the presence of a by-produced polymer.

2. Description of the Prior Art

Linear α-olefins are linear or non-branched olefins, each of which has an even number (4 or more) of carbon atoms and has a carbon-carbon double bond at its terminal. In particular, linear α-olefins having 4 - 18 carbon atoms are useful chemical compounds widely used as a monomer for olefin polymer, as a co-monomer for various kinds of high molecular polymer or as a material for plasticizer and surface active agent.

Heretofore, the linear α-olefins have been produced by oligomerizing a lower olefin such as ethylene as a raw material in the presence of a Ziegler-type catalyst. When the linear α-olefins are produced by oligomerizing a lower olefin in the presence of a Ziegler-type catalyst, a polymer with the polymerization degree of 1000 to 100000 is produced as a by-product. Although a technique of separating the by-produced polymer from the system after the oligomerization reaction or after the deactivation of the catalyst may be employed, it is very complex in equipment, and undesirable. Therefore, a technique of separating the by-produced polymer from the bottom of a distillation tower after separating linear α-olefins by distillation has been employed. However, when the reaction mixture containing the by-produced polymer together with linear α-olefins is subjected to distillation treatment, the catalyst, which has been contained in the by-produced polymer, and its metal components produced after the deactivation of the catalyst are brought into contact with the linear α-olefins contained in the reaction mixture due to the melting of the by-produced polymer at the bottom of the distillation tower. At this time, a polymer is further produced as a by-product, so that the recovery rate of the linear α-olefins is lowered. In addition, because of the contact of the linear α-olefins with un-deactivated catalyst, the reaction of the linear α-olefins themselves takes place, whereby the purity of a linear α-olefin product is lowered. Further, the accumulation or deposition of the catalyst and its metal components will bring a problem in blockade of the distillation tower.

Therefore, a treatment of removing the catalyst and its metal components from the reaction mixture, in particular, from the by-produced polymer contained in the reaction mixture, that is, an ash removing treatment, is required before the distillation treatment.

Various techniques are known as an ash removing method. For example, a technique using an alcohol as an ash removing agent is known. In this technique, the catalyst and its metal components can be removed by swelling the by-produced polymer with the alcohol or the like even in the case where the by-produced polymer exists. That is, this technique is excellent in ash removing efficiency. However, the alcohol as an ash removing agent is soluble in an oligomerization solvent, so that it is difficult to separate the alcohol from the linear α-olefins and the oligomerization solvent. Therefore, special equipment for separation and recovery of the ash removing agent is required, and there arises a problem in that equipment cost in production apparatus becomes large.

On the other hand, an technique of removing an ash content at a relatively low temperature using an ash removing agent such as water which is insoluble in the oligomerization solvent is also known (see for example EP A 0 320 571). In this technique, the ash removing agent can be separated easily. Accordingly, this technique has an advantage in that there is no trouble in separation and recovery of the ash removing agent. However, in this technique, the ash removing rate is low. In particular, when an ash removing treatment is conducted in the presence of the by-produced polymer which contains the catalyst and its metal components, the catalyst and the metal components cannot be removed perfectly without execution of a special pre-treatment such as a by-produced polymer grinding treatment.

An object of the present invention is to provide a process of producing linear α-olefins in which when the linear α-olefins are produced by oligomerizing a lower olefin in the presence of a Ziegler-type catalyst, not only the catalyst and its metal components can be removed to a very low concentration in the presence of a by-produced polymer but also an ash removing agent can be separated and recovered easily with no special consideration.

Other objects of the present invention will be apparent from the following descriptions and the drawings.

SUMMARY OF THE INVENTION

The inventors have investigated various ash removing treatments to attain the foregoing objects. As a result, it has been found that not only excellent ash removing rate can be attained even in the presence of a by-produced polymer but also the ash removing agent can be removed easily, by stirring a reaction mixture together with a predetermined quantity of water as an ash removing agent at a predetermined temperature and under a predetermined amount of power. The present invention has been completed based on this finding.

That is, the present invention provides a process of producing linear $\alpha$-olefins which comprises the steps of: oligomerizing a lower olefin in the presence of a Ziegler-type catalyst; deactivating the catalyst after oligomerization; adding water to a linear $\alpha$-olefins-containing reaction mixture, in a weight ratio of water to the organic phase in the reaction mixture of 0.2 or above; and stirring the resulting mixture at a temperature of 90°C or above and under a power of 3KW/m$^3$ or above per liquid unit volume to thereby remove an ash content.

BRIEF DESCRIPTION OF THE DRAWINGS

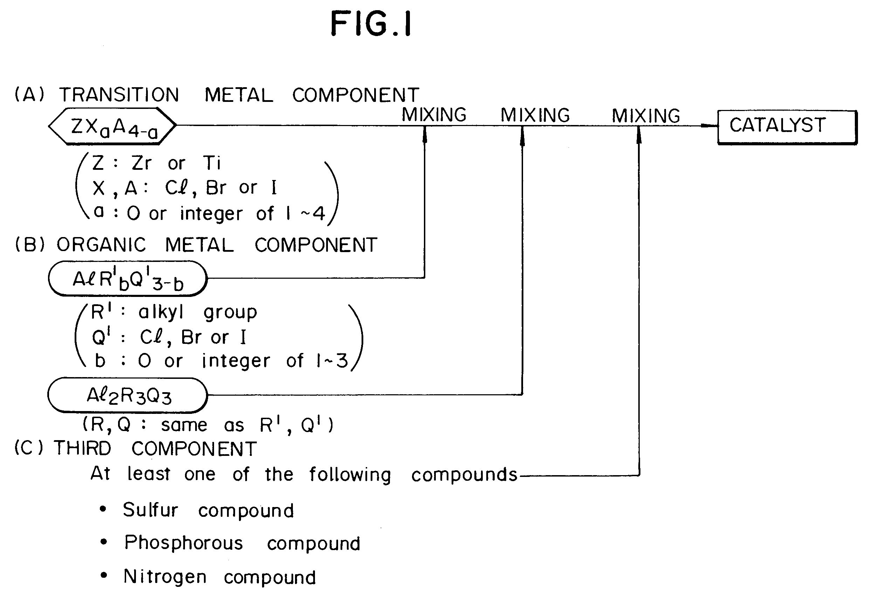

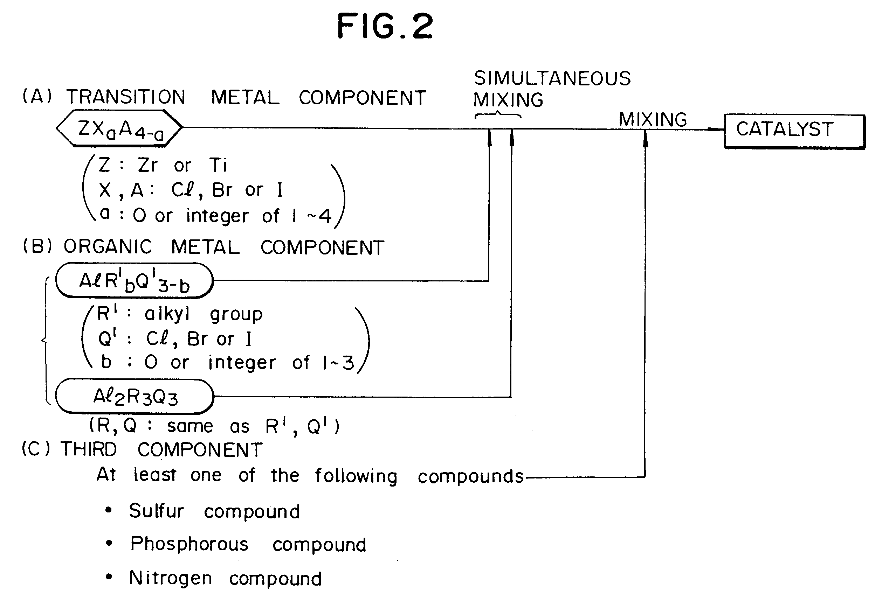

Figs. 1 and 2 are flow sheets showing preparation of a catalyst.

DETAILED DESCRIPTION OF THE INVENTION

The present invention will be described hereinbelow in detail.

In the process according to the present invention, linear $\alpha$-olefins are produced by oligomerizing a lower olefin in the presence of a Ziegler-type catalyst. As the lower olefin, ethylene is usually used. As shown in Fig. 1 and Fig. 2, the Ziegler-type catalyst is prepared by mixing a transition metal component (A), an organic metal component (B) and an optionally employed third component (C). As the transition component (A), there is used a chemical compound represented by the general formula:

$$ZX_aA_{4-a} \quad (I)$$

in which Z represents zirconium or titanium, each of X and A represents chlorine, bromine or iodine (X and A may be the same or different from each other), and $\underline{a}$ represents 0 or an integer from 1 through 4. Examples of the chemical compound include $ZrCl_4$, $ZrBr_4$, $ZrI_4$, $ZrBrCl_3$, $ZrBr_2Cl_2$, $TiCl_4$, $TiBr_4$, $TiI_4$, $TiBrCl_3$, $TiBr_2Cl_2$, etc. Among these compounds, $ZrCl_4$ is particularly preferred. These compounds may be used singly or in combination.

The organic metal component (B) includes a chemical compound represented by the general formula:

$$Al_2R_3Q_3 \quad (II)$$

(in which R represents alkyl group having 1 - 20 carbon atoms, and Q represents chlorine, bromine or iodine) and/or a chemical compound represented by the general formula:

$$AlR^1_bQ^1_{3-b} \quad (III)$$

(in which $R^1$ represents alkyl group having 1 - 20 carbon atoms, $Q^1$ represents chlorine, bromine or iodine, $\underline{b}$ represents 0 or an integer of 1 through 3).

Examples of the chemical compound represented by the general formula (II) include
$Al_2(CH_3)_3Cl_3$,
$Al_2(CH_3)_3Br_3$,
$Al_2(C_2H_5)_3Cl_3$,
$Al_2(C_2H_5)_3Br_3$,
$Al_2(C_2H_5)_3I_3$,
$Al_2(C_2H_5)_3BrCl_2$,
$Al_2(C_3H_7)_3Cl_3$,
$Al_2(iso-C_3H_7)_3Cl_3$,
$Al_2(C_4H_9)_3Cl_3$,
$Al_2(iso-C_4H_9)_3Cl_3$,
$Al_2(C_5H_{11})_3Cl_3$,

3

$Al_2(C_8H_{17})_3Cl_3$,

$Al_2(C_2H_5)_2(CH_3)Cl_3$, etc.

Examples of the chemical compound represented by the general formula (III) include

$Al(CH_3)_3$, $Al(C_2H_5)_3$,

$Al(C_3H_7)_3$, $Al(iso-C_3H_7)_3$,

$Al(C_4H_9)_3$, $Al(iso-C_4H_9)_3$,

$Al(C_5H_{11})_3$, $Al(C_6H_{13})_3$,

$Al(C_8H_{17})_3$, $Al(C_2H_5)_2Cl$,

$Al(C_2H_5)_2Br$, $Al(C_2H_5)_2I$,

$Al(C_2H_5)Cl_2$, $Al(C_2H_5)Br_2$,

$Al(C_2H_5)I_2$, etc.

The third component which is optionally used is at least one compound selected from sulfur compounds, phosphorus compounds and nitrogen compounds.

Any sulfur compound can be used as the third component as long as it is an organic sulfur compound. In general, examples of the sulfur compound preferably used include: dialkyl or diaryl sulfides (thioethers) such as dimethyl sulfide, diethyl sulfide, dipropyl sulfide, dihexyl sulfide, dicyclohexyl sulfide, diphenyl sulfide, etc.; dialkyl disulfides such as dimethyl disulfide, diethyl disulfide, dipropyl disulfide, dibutyl disulfide, dihexyl disulfide, dicyclohexyl disulfide, ethylmethyl disulfide, etc.; heterocyclic sulfur compounds such as thiophenes (e.g. thiophene, 2-methylthiophene, 3-methylthiophene, 2,3-dimethylthiophene, 2-ethylthiophene, benzo-thiophene), tetrahydrothiophene, thiopyran, etc.; aromatic sulfur compounds such as diphenyl sulfide, diphenyl disulfide, methylphenyl disulfide, methylphenyl sulfide, etc.; thiourea; and sulfide compounds such as methyl sulfide, ethyl sulfide, butyl sulfide, etc.

Any phosphorus compound can be used as the third component as long as it is an organic phosphorus compound. In general, examples of the phosphorus compound preferably used include phosphine compounds such as triphenyl phosphine, triethyl phosphine, tributyl phosphine, tripropyl phosphine, trioctyl phosphine, tricylohexyl phosphine, etc.

Any nitrogen compound can be used as the third component as long as it is an organic nitrogen compound. In general, examples of the nitrogen compound preferably used include organic amine compounds such as methylamine, ethylamine, propylamine, butylamine, pentylamine, hexylamine, cyclohexylamine, octylamine, decylamine, aniline, benzylamine, naphthylamine, dimethylamine, diethylamine, dibutylamine, diphenylamine, methylphenylamine, trimethylamine, triethylamine, tributylamine, triphenylamine, pyridine, picoline, etc.

Among these sulfur compounds, phosphorus compounds and nitrogen compounds, at least one kind of compound selected from dimethyl disulfide, thiophene, thiourea, triphenyl phosphine, tributyl phosphine, trioctyl phosphine, aniline, etc. can be used as the preferred compound.

Using the aforementioned components (A), (B) and (C), a Ziegler-type catalyst solution is prepared as follows. As shown in Fig. 1, $AlR^1_bQ^1_{3-b}$ in the organic metal component (B) or an organic solvent solution thereof is mixed with an organic solvent solution of $ZX_aA_{4-a}$ as the transition metal component (A). Then, the resulting mixture is mixed with $Al_2R_3Q_3$ in the organic metal component (B) or an organic solvent solution thereof. Further, a sulfur, phosphorus or nitrogen compound as the third component (C) is added into the resulting mixture to thereby prepare a catalyst solution. Another catalyst solution preparing method may be used. As shown in Fig. 2, $AlR^1_bQ^1_{3-b}$ and $Al_2R_3Q_3$ as the organic metal component (B) or an organic solvent solution thereof is mixed with an organic solvent solution of $ZX_aA_{4-a}$ as the transition metal component (A). Then, the resulting mixture is mixed with a sulfur, phosphorus or nitrogen compound as the third component (C) to thereby prepare a catalyst solution.

The organic solvent used in the catalyst solution may be directly used as a solvent for oligomerizing a lower olefin. Examples of the organic solvent used herein include: naphthenic paraffins such as cyclohexane, decaline, etc.; aromatic hydrocarbons which may be substituted with halogen atom(s) such as benzene, toluene, xylene, chlorobenzene, ethylbenzene, dichlorobenzene, chlorotoluene, etc.; aliphatic paraffins such as pentane, hexane, heptane, octane, nonane, decane, etc.; and haloalkanes such as dichloroethane, dichlorobutane, etc. Among them, the preferred are cyclohexane and benzene.

According to the present invention, the components (A), (B) and (C) are used in the following proportions. The amount of the component (A) is in general 0.01 to 5 millimoles, preferably 0.03 to 1 millimole per 250 ml of the solvent. The amount of the component (B) is in general 0.05 to 15 millimoles, preferably 0.06 to 3 millimoles, per 250 ml of the solvent. The amount of the component (C) is in general 0.05 to 20 millimoles per 250 ml of the solvent. In the case where a sulfur compound as defined above is used as the component (C), the preferred amount of the component (C) is 0.1 to 10 millimoles. In the case where a nitrogen or phosphorus compound as defined above is used as the component (C), the preferred amount of the component (C) is 0.05 to 5 millimoles. In respect to the mixing ratio of the components (A) and (B), a more preferred result can be attained by defining Al/Zr (mole ratio) within a range of 1/1 to 15/1.

In general, the oligomerization of an olefin is carried out at a temperature of 100 to 130°C and under a pressure of 30 to $70kg/cm^2 \cdot G$. The reaction time cannot be determined simply because it is influenced by temperature and pressure. In general, the sufficient reaction time is about 10 to about 60 minutes.

In the present invention, a reaction mixture prepared after the olefin oligomerization is subjected to catalyst deactivation treatment. It is preferable that the deactivation treatment is carried out as follows. A reaction mixture from an oligomerization reactor is transferred to a catalyst deactivation tank. In the catalyst deactivation tank, the reaction mixture is subjected to deactivation treatment by adding a catalyst deactivating agent such as amine, aqueous ammonia, etc. thereto and stirring them at a predetermined temperature for a predetermined time.

In the present invention, ash removing treatment is carried out after the catalyst deactivation treatment. The ash removing treatment is the crux of the present invention and is carried out as follows. First, water is added to a reaction mixture containing linear $\alpha$-olefins. The amount of water added is defined to be 0.2 time or above (in terms of weight) the amount of the organic phase in the reaction mixture. This is because when it is less than 0.2 time, the catalyst and its metal components cannot be sufficiently dissolved in water so that ash removing rate is lowered, while when it is 0.2 time or above, they can be sufficiently dissolved in water so that ash removing rate is increased. When water is used in a larger amount, it is of course possible to conduct the ash removal without trouble, but a volume of the liquid to be treated thereafter is increased. Therefore, it is preferable that the amount of water is about 0.5 time or below the amount of the organic phase in the reaction mixture. Particularly, the preferred amount of water is 0.2 time to 0.5 time. It is more preferable that alkali hydroxide such as sodium hydroxide is added simultaneously with the addition of water. This is because the precipitation of aluminum hydroxide can be prevented by the addition of the strong alkali compound.

After water is added, the resulting mixture is stirred while heating. The heating is conducted at a temperature of 90°C or above. The reason is as follows. If the temperature is less than 90°C, the by-produced solid polymer cannot be softened nor melted so that the catalyst and its metal components contained in the by-produced polymer cannot be brought into contact with water sufficiently. As a result, ash removing rate is lowered. On the contrary, when the temperature is 90°C or above, the by-produced solid polymer is softened or melted so that the catalyst and its metal components contained in the by-produced polymer can be brought into contact with water as an ash removing agent. As a result, ash removing rate is improved. The preferred heating temperature is 90 to 150 °C.

The stirring under the heating is performed under a stirring power of 3 $KW/m^3$ or above per liquid unit volume. The reason is as follows. If the power is less than 3 $KW/m^3$, the contacting area between the reaction mixture and water as an ash removing agent is so small that ash removing rate is lowered. When the power is 3 $KW/m^3$ or above, the contacting area between the reaction mixture and water is so large that ash removing rate is improved. The preferred power required for stirring is 4 to 10 $KW/m^3$.

As described above, the ash removing treatment in the present invention has the following essential conditions.

(a) The amount of water added is 0.2 time or above (in terms of weight) the amount of the organic phase in the reaction mixture.

(b) The heating temperature is 90°C or above.

(c) The power required for stirring is 3 $KW/m^3$ or above.

If one or two are employed among the aforementioned conditions (a), (b) and (c), ash removing rate can be improved to some degree. However, in the present invention, all of the conditions (a), (b) and (c) are employed to attain a high ash removing rate.

By adding a defined amount of water, heating the resulting mixture at a defined temperature and stirring it under a defined power as described above, the catalyst and its metal components can be removed to a very low concentration so that the ash removing treatment can be conducted very efficiently. Although the heating/stirring time is not critical, a short time of 1 to 10 minutes may be used generally. Although the time for standing the mixture after the heating/stirring is not critical, a short time of 10 to 30 minutes may be used generally.

After standing the mixture as it is, the mixture is separated into the organic phase and the water phase. The organic phase is distillated to separate and recover linear $\alpha$-olefins having 4 or more carbon atoms, in particular, having 4 to 18 carbon atoms, as a distillate. On the other hand, the by-produced polymer as a residue is discharged from the bottom of the distillation tower.

According to the present invention, the catalyst and its metal components have been removed to a very low concentration by the aforementioned ash removing treatment. Accordingly, when the organic phase is subjected to distillation, linear $\alpha$-olefins are prevented from further polymerizing during the distillation to by-produce a polymer. In addition, a side reaction is also prevented. Therefore, linear $\alpha$-olefins as a desired product can be produced in high yield, while maintaining high purity of the linear $\alpha$-olefins. In addition, there is no trouble of blockade in the distillation tower by the catalyst and its metal components. Accordingly, the present invention has an industrial significance.

The following examples illustrate the present invention more in detail, though the invention is not limited to these examples.

Example 1

⟨ Preparation of Catalyst Solution ⟩

In 500 ml of a flask with a stirrer, 25 mmol (millimoles) of anhydrous zirconium tetrachloride ($ZrCl_4$) and 250 ml of dried cyclohexane were introduced under an argon atmosphere. The mixture (100 mmol of $ZrCl_4$ per 1 litre of cyclohexane) was stirred at room temperature for 10 minutes. Triethyl aluminum [$(C_2H_5)_3Al$] and ethyl aluminum sesquichloride [$(C_2H_5)_3Al_2Cl_3$] were successively added in this order to the mixture.

The amount of triethyl aluminum and ethyl aluminum sesquichloride was determined to satisfy the following relations.

$(C_2H_5)_3Al_2Cl_3/(C_2H_5)_3Al$ = 3.5 (molar ratio)
$\{(C_2H_5)_3Al + (C_2H_5)_3Al_2Cl_3\}/ZrCl_4$ = 7 (molar ratio)

After the stirring was started, the mixture was heated at 70°C under an argon atmosphere to form a complex. Thus, a complex solution is prepared.

Then, the complex solution and cyclohexane dried under the argon atmosphere were mixed in predetermined amounts to prepare a mixture (0.5 mmol $ZrCl_4$, 0.78 mmol $(C_2H_5)_3Al$ and 2.72 mmol $(C_2H_5)_3Al_2Cl_3$ per 1 litre of cyclohexane). Thiophene was added to the mixture in a molar ratio of thiophene/$ZrCl_4$ = 3/1 to prepare a catalyst solution.

⟨ Oligomerization ⟩

The oligomerization of ethylene was continuously carried out using a complete mixing tank type reactor (inner volume: 1 litre). That is, the catalyst solution prepared as described above was supplied to the reactor at a constant rate (700 cc per hour) and simultaneously high purity ethylene is supplied to the reactor so that reaction pressure was kept to be 65 kg/cm$^2$ · G to oligomerize the ethylene. The residence time in the reactor was 30 minutes on solvent base. The reaction temperature was 120°C.

The oligomerization reaction mixture thus prepared contained linear $\alpha$-olefins, by-produced polymer and catalyst. The linear $\alpha$-olefins had the following distribution.

| Linear $\alpha$-olefin | Distribution (%) |
|---|---|
| $C_4$ | 15.1 |
| $C_6$ | 15.5 |
| $C_8$ | 14.2 |
| $C_{10}$ | 12.1 |
| $C_{12}$ | 10.0 |
| $C_{14}$ | 8.0 |
| $C_{16}$ | 6.2 |
| $C_{18}$ | 4.8 |
| $\geqq C_{20}$ | 14.1 |

〈 Deactivation Treatment 〉

The reaction mixture from the reactor was transferred to a catalyst deactivation tank, in which 30% aqueous ammonia was continuously supplied to the reaction mixture. The amount of 30% aqueous ammonia supplied was 10% by volume of the reaction mixture. Thereafter the mixture was stirred at 40°C to deactivate the catalyst.

〈 Ash Removing Treatment 〉

The reaction mixture which has been subjected to the deactivation treatment as described above was further subjected to the following ash removing treatment.

The reaction mixture was put into 3000 ml of a cylindrical glass vessel having a thermometer, a condenser, four baffle plates and four fan turbine wings. An aqueous sodium hydroxide solution (pH = 12) in an amount of 0.3 time (in terms of weight) the amount of the organic phase in the reaction mixture was added to the reaction mixture. The resulting mixture was heated at 100°C and stirred under a power of 5 $kW/m^3$ per unit volume for 5 minutes. After the stirring was stopped, the mixture was settled for 20 minutes to separate an organic phase and a water phase. Then, the organic phase was taken out without disturbance of liquid-liquid interface, and the metal components contained in the organic phase was measured. The ash removing rate was calculated in the following equation.

$$\text{Ash removing rate (\% by weight)} = \frac{A - B}{A} \times 100$$

A : Metal content in the reaction mixture before ash removing treatment (ppm by weight)
B : Metal content in the organic phase after ash removing treatment (ppm by weight)

The condition for the ash removing treatment and the result thereof in Example 1 were shown in Table 1.

As is obvious from Table 1, this example gave a good result that the ash removing rates of Zr and Al are 96.0 % and 98.2 %, respectively.

Examples 2 to 5 and Comparative Examples 1 to 6

Example 1 was repeated except that the condition for ash removing treatment was changed variously as shown in Table 1.

As a result, as is obvious from Table 1, Examples 2 to 5 in which all of the added water amount, heating temperature and stirring power satisfy the values defined in the present invention gave the same good results as Example 1. On the contrary, Comparative Examples 1 to 6 in which at least one of the three conditions does not satisfy the values defined in the present invention did not give the desired ash removing effect.

TABLE 1

| | | Ex. 1 | Ex. 2 | Ex. 3 | Ex. 4 | Ex. 5 | Cmp. 1 | Cmp. 2 | Cmp. 3 | Cmp. 4 | Cmp. 5 | Cmp. 6 |
|---|---|---|---|---|---|---|---|---|---|---|---|---|
| Metal content in reaction mixture | Zr (mg/l) | 7.0 | 7.0 | 7.0 | 7.0 | 7.0 | 7.0 | 7.0 | 7.0 | 7.0 | 7.0 | 7.0 |
| | Al (mg/l) | 26.0 | 26.0 | 26.0 | 26.0 | 26.0 | 26.0 | 26.0 | 26.0 | 26.0 | 26.0 | 26.0 |
| Ash removing conditions | Water phase/organic phase (W/O) (wt/wt) | 0.3 | 0.3 | 0.3 | 0.3 | 0.3 | 0.3 | 0.3 | 0.1 | 0.3 | 0.3 | 0.3 |
| | power per unit volume (PV) ($KW/m^3$) | 5 | 5 | 5 | 5 | 8 | 5 | 5 | 5 | 3 | 1 | 1 |
| | Heating temperature (°C) | 100 | 110 | 120 | 130 | 100 | 80 | 40 | 40 | 40 | 40 | 100 |
| | Heating/stirring time (min) | 5 | 5 | 5 | 5 | 5 | 5 | 5 | 5 | 5 | 5 | 5 |
| | Standing time (min) | 20 | 20 | 20 | 20 | 20 | 20 | 20 | 20 | 20 | 20 | 20 |
| Ash removing rate (%) | Zr | 96.0 | 97.1 | 98.0 | 98.9 | 97.2 | 89.8 | 76.1 | 72.9 | 71.8 | 61.5 | 89.3 |
| | Al | 98.2 | 99.6 | 99.8 | 99.8 | 99.6 | 95.2 | 87.3 | 82.3 | 80.4 | 73.4 | 94.9 |

According to the present invention, when linear α-olefins are produced by oligomerizing a lower olefin using a Ziegler-type catalyst, not only the catalyst and its metal components can be removed to a very low concentration in the presence of a by-produced polymer but also an ash removing agent can be separated and recovered easily.

As a result, linear α-olefins excellent in product quality can be produced without blockade of a distillation tower during the distillation of the linear α-olefins.

The linear α-olefins produced by the process according to the present invention are useful as a monomer for olefin polymer or a co-monomer for various kinds of high molecular polymer or as a material for plasticizer, surface active agent, etc. and greatly contributes to these fields.

**Claims**

1. A process of producing linear α-olefins which comprises the steps of: oligomerizing a lower olefin in the presence of a Ziegler-type catalyst; deactivating said catalyst after oligomerization; adding water to a linear α-olefins-containing reaction mixture, in a weight ratio of water to the organic phase in said reaction mixture of 0.2 or above; and stirring the resulting mixture at a temperature of 90°C or more and under a power of $3KW/m^3$ or above per liquid unit volume to thereby remove an ash content.

2. A process according to claim 1, wherein said lower olefin as a raw material is ethylene.

3. A process according to claim 1, wherein said linear α-olefins as a desired product are linear α-olefins having 4 to 18 carbon atoms.

4. A process according to claim 1, wherein the weight ratio of the water to the organic phase is 0.2 time to 0.5 time.

5. A process according to claim 1, wherein alkali hydroxide is added simultaneously with the addition of water.

6. A process according to claim 1, wherein the heating temperature is 90 to 150 °C.

7. A process according to claim 1, wherein the power per unit volume is 4 to 10 $KW/m^3$.

**Patentansprüche**

1. Verfahren zur Herstellung linearer α-Olefine, das die Schritte umfaßt: Oligomerisieren eines niederen Olefins in Gegenwart eines Zieglerkatalysators; Desaktivieren des genannten Katalysators nach dem Oligomerisieren; Zugeben von Wasser zu der die linearen α-Olefine enthaltenden Reaktionsmischung in einem Gewichtsverhältnis von Wasser zur organischen Phase in der genannten Reaktionsmischung von 0,2 oder darüber; und Rühren der resultierenden Mischung bei einer Temperatur von 90°C oder mehr und mit einer Kraft von 3 $kW/m^3$ oder darüber pro Flüssigkeitsvolumeneinheit, um dabei den Aschegehalt zu entfernen.

2. Verfahren nach Anspruch 1, worin das genannte niedere Olefin als Rohprodukt Ethylen ist.

3. Verfahren nach Anspruch 1, worin die genannten linearen α-Olefine als gewünschte Produkte lineare α-Olefine mit 4 bis 18 Kohlenstoffatomen sind.

4. Verfahren nach Anspruch 1, worin das Gewichtsverhältnis von Wasser zur organischen Phase 0,2 bis 0,5 beträgt.

5. Verfahren nach Anspruch 1, worin gleichzeitig mit der Zugabe von Wasser Alkalihydroxid hinzugegeben wird.

6. Verfahren nach Anspruch 1, worin die Heiztemperatur 90 bis 150°C beträgt.

7. Verfahren nach Anspruch 1, worin die Kraft pro Volumeneinheit 4 bis 10 $kW/m^3$ beträgt.

**Revendications**

1. Procédé de production d'α-oléfines linéaires selon lequel on oligomérise une oléfine inférieure en présence d'un catalyseur de type Ziegler, on désactive ce catalyseur après oligomérisation ; on ajoute de l'eau dans un mélange réactionnel contenant des $\alpha$-oléfines linéaires, selon un rapport pondéral de l'eau à la phase organique dans le mélange réactionnel de 0,2 ou plus ; et on agite le mélange résultant à une température de 90 °C ou plus, selon une puissance de 3 kW/m$^3$ ou plus par volume unitaire de liquide afin d'éliminer les cendres contenues.

**2.** Procédé selon la revendication 1, dans lequel l'oléfine inférieure formant matière première est de l'éthylène.

**3.** Procédé selon la revendication 1, dans lequel les $\alpha$-oléfines linéaires formant un produit requis, comprennent des $\alpha$-oléfines linéaires comportant de 4 à 18 atomes de carbone.

**4.** Procédé selon la revendication 1, dans lequel le rapport pondéral de l'eau à la phase organique est de 0,2 à 0,5 fois.

**5.** Procédé selon la revendication 1, dans lequel l'hydroxyde alcalin est ajouté en même temps que l'eau est additionnée.

**6.** Procédé selon la revendication 1, dans lequel la température de chauffage est de 90 à 150 °C.

**7.** Procédé selon la revendication 1, dans lequel la puissance par volume unitaire est de 4 à 10 kW/m$^3$.

# FIG.1

(A) TRANSITION METAL COMPONENT

$ZX_aA_{4-a}$

$$\left(\begin{array}{l} Z : Zr\ or\ Ti \\ X,\ A : Cl,\ Br\ or\ I \\ a : O\ or\ integer\ of\ 1\sim4 \end{array}\right)$$

(B) ORGANIC METAL COMPONENT

$Al\,R^I_b Q^I_{3-b}$

$$\left(\begin{array}{l} R^I : alkyl\ group \\ Q^I : Cl,\ Br\ or\ I \\ b : O\ or\ integer\ of\ 1\sim3 \end{array}\right)$$

$Al_2R_3Q_3$

$(R,\ Q : same\ as\ R^I,\ Q^I)$

(C) THIRD COMPONENT

At least one of the following compounds

- Sulfur compound
- Phosphorous compound
- Nitrogen compound

MIXING → MIXING → MIXING → CATALYST

# FIG.2

(A) TRANSITION METAL COMPONENT

SIMULTANEOUS MIXING

MIXING

CATALYST

$ZX_aA_{4-a}$

$$\begin{pmatrix} Z:\ Zr\ or\ Ti \\ X,A:\ C\ell,\ Br\ or\ I \\ a:\ 0\ or\ integer\ of\ 1\sim4 \end{pmatrix}$$

(B) ORGANIC METAL COMPONENT

$A\ell R^1{}_b Q^1{}_{3-b}$

$$\begin{pmatrix} R^1:\ alkyl\ group \\ Q^1:\ C\ell,\ Br\ or\ I \\ b:\ 0\ or\ integer\ of\ 1\sim3 \end{pmatrix}$$

$A\ell_2 R_3 Q_3$

(R, Q : same as $R^1$, $Q^1$)

(C) THIRD COMPONENT

At least one of the following compounds

• Sulfur compound

• Phosphorous compound

• Nitrogen compound

EP 0 444 505 B1